# EUROPÄISCHE PATENTANMELDUNG

(11) **EP 1 190 736 A2**
(43) Veröffentlichungstag der Anmeldung: **27.03.2002**
(21) Anmeldenummer: 01250336.3
(22) Anmeldetag: 25.09.2001
(51) Int. Cl.: A61N 1/378

(54) **Elektrisch aktives medizinisches implantat**

(30) Priorität: 26.09.2000 DE 10048648
(71) Anmelder: BIOTRONIK Mess- und Therapiegeräte GmbH & Co Ingenieurbüro Berlin, 12359 Berlin (DE)
(72) Erfinder: Digby, Dennis, Wilsonville, OR 97070 (US); Schaldach, Max, (DE)
(74) Vertreter: Eisenführ, Speiser & Partner

(57) **Zusammenfassung**

Elektrisch aktives medizinisches Implantat, mit einer Schaltung (5), mindestens einer ersten Batterie (1) zum Abgeben eines niedrigen Stromes und einer zweiten Batterie (2) zum Abgeben eines hohen Stromes an die Schaltung (5), und einem Steuermittel (4) zum Abkoppeln der ersten Batterie (1) von der Schaltung (5) und zum Ankoppeln der zweiten Batterie (2) an die Schaltung (5), wobei ein parallel zu der ersten Batterie (1) geschalteter Kondensator (3), der von der ersten Batterie während eines ersten Schaltzustandes, während dem die erste Batterie von der Schaltung (5) angekoppelt ist und während eines zweiten Schaltzustandes, während dem die Schaltung (5) nur an der zweiten Batterie (1) angekoppelt ist, aufladbar ist, wobei das Steuermittel (4) die zweite Batterie (2) am Ende des zweiten Schaltungszustandes wieder von der Schaltung (5) abkoppelt und die Parallelschaltung aus der ersten Batterie (1) und dem Kondensator (3) zur weiteren Energieversorgung wieder an die Schaltung (5) ankoppelt.

## Beschreibung

Die vorliegende Erfindung betrifft ein elektrisch aktives medizinisches Implantat mit einer Schaltung, mindestens einer ersten Batterie zum Abgeben eines niedrigen Stromes und einer zweiten Batterie zum Abgeben eines hohen Stromes an die Schaltung, und einem Steuermittel zum Abkoppeln der ersten Batterie von der Schaltung und zum Ankoppeln der zweiten Batterie an die Schaltung.

Bei medizinischen Implantaten, insbesondere bei Defibrillatoren werden üblicherweise zwei Batterien als Energieversorgung verwendet. Die erste Batterie ist dabei als eine Batterie mit einer niedrigen Rate (die Rate entspricht hierbei der Höhe der Lade- oder Entladestrommenge, bezogen auf die Kapazität der Batterie, in einer bestimmten Zeit) und mit einer hohen Speicherkapazität ausgestaltet. Die zweite Batterie ist dagegen als Batterie mit einer hohen Rate und mit einer geringeren Speicherkapazität ausgestaltet. Für die normalen Überwachungen und Schrittmacherfunktionen, welche einen Strom in der Größenordnung von einigen 10 µA benötigen, wird die Batterie mit niedriger Rate verwendet, während die Batterie mit einer hohen Rate für Aktivitäten wie beispielsweise den Betrieb eines Mikroprozessors und für Therapieanwendungen zum Einsatz kommt. Typischerweise liegt der benötigte Strom während derartiger Aktivitäten mit einer hohen Rate im Bereich von 200 µA bis 1,5 A.

Typischerweise ist die Batterie mit einer niedrigen Rate als eine Lithium-Jodid (Lil) Batterie ausgestaltet, während eine Batterie mit einer hohen Rate typischerweise als eine Lithium-Silber-Vanadiumoxid (SVO)- oder als eine Lithium-Manganoxid Batterie ausgestaltet ist. Die Energiedichte (mAh pro cm³) einer Lil Batterie, d.h. eine Batterie mit einer niedrigen Rate, ist typischerweise doppelt so hoch wie die einer Batterie mit einer hohen Rate.

Da Aktivitäten mit einer hohen Rate jedoch selten sind, wird die Batterie mit der niedrigen Rate vorwiegend verwendet. Üblicherweise wird zu der Batterie mit der hohen Rate umgeschaltet, wenn die Spannung der Batterie mit der niedrigen Rate aufgrund des Innenwiderstandes der Batterie unter eine vorbestimmte Spannung fällt.

Die Langlebigkeit bzw. die Lebensdauer des Implantates wird durch Addition der Lebensdauer der Batterie mit der niedrigen Rate mit der Lebensdauer der Batterie mit der hohen Rate bestimmt. Dies gilt jedoch nur für die Überwachungsfunktion. Wenn eine Therapie oder andere Aktivitäten mit einer hohen Rate benötigt werden, muss die während dieser Aktivität mit einer hohen Rate verwendete Strommenge von der Kapazität der Batterie mit der hohen Rate zur Bestimmung der Lebensdauer der Batterie mit der hohen Rate herangezogen werden.

In jeder Batterie ist immer mehr chemische Energie vorhanden als durch eine Schaltung extrahiert werden kann. Mit zunehmender Erschöpfung der Batterie steigt der Innenwiderstand bis zu einem Punkt, an dem der Spannungsabfall an dem Innenwiderstand (Strom x Widerstand) bewirkt, dass die Ausgangsspannung der Batterie unter den minimal für die Schaltung verwendbaren Spannungsschwellwert fällt.

Aus der US 6,044,295 ist ein medizinisches Implantat bekannt, welches eine erste Batterie für Komponenten, die einen niedrigen Strom benötigen, und eine zweite Batterie für Komponenten, die einen großen Strom benötigen, sowie eine Schaltvorrichtung aufweist. Die Schaltvorrichtung koppelt die zweite Batterie lediglich solange an eine zweite Therapieeinheit, wie ein Steuersignal ausgegeben wird, das anzeigt, dass ein vorbestimmter Spannungspegel der ersten Batterie nicht erreicht wird oder dass der Innenwiderstand einen spezifischen Wert überschritten hat. Die Schaltvorrichtung verbindet die zweite Batterie dann auch mit einer ersten Therapieeinheit.

US 6,008,625 offenbart ferner ein medizinisches Implantat mit zwei Batterien. Die erste Batterie betreibt dabei das medizinische Implantat sowohl in einer Überwachungsbetriebsart beispielsweise zum Überwachen der Herzfrequenz, als auch in einer Betriebsart zum Aufladen von Kondensatoren, welche eine elektrische Entladung mit einer hohen Rate benötigen. Wenn die erste Batterie bis zu einem vorbestimmten Spannungspegel entladen ist, wird die erste Batterie lediglich für die Überwachungsfunktion verwendet. Ab diesem Zeitpunkt wird die zweite Batterie auch für elektrische Pulsentladungen mit einer hohen Rate verwendet. Wenn die erste Batterie bis zu einem vorbestimmten Maße erschöpft ist, übernimmt die zweite Batterie sowohl die Überwachung des Implantates als auch die anderen Betriebsfunktionen.

Bei beiden oben genannten Druckschriften wird eine Batterie von der restlichen Schaltung abgekoppelt, wenn ihre Ausgangsspannung unter einen vorgegebenen Spannungspegel fällt. Nachteilig erweist es sich dabei, dass die sich in der Batterie zu diesem Zeitpunkt befindliche Energie dann nicht weiter genutzt wird.

Aus der US 5,814,075 ist ein ICD (implantierbarer Cardioverter/Defibrillator) bekannt, der zwei unterschiedliche Batterien aufweist, zwischen denen entsprechend eines Satzes von Fuzzy-Regeln umgeschaltet wird. Die Regeln sollen so gewählt sein, dass beide Batterien optimal ausgenutzt werden und eine Explantation nicht allein deshalb durchgeführt werden muss, weil nur eine der beiden Batterien erschöpft ist. Nachteilig ist die aufwendige Steuerung für das Umschalten.

Aus der EP 0 771 576 ist ein ICD bekannt, bei dem Ausgangsspannungen zweier Batterien über verschiedene Spannungsbusse einer Steuerung zugeführt sind.

Es ist eine Aufgabe der Erfindung, ein alternatives medizinisches Implantat vorzusehen, welches die sich in ihren Batterien befindliche Energie weitestgehend ausnützt.

Die Aufgabe der vorliegenden Erfindung wird durch ein elektrisch aktives medizinisches Implantat gemäß dem Anspruch 1 gelöst.

Der Erfindung liegt dabei der Gedanke zugrunde, ein elektrisch aktives medizinisches Implantat mit einer Schaltung, einer ersten und zweiten Batterie zum Abgeben eines niedrigen Stromes und eines hohen Stromes an die Schaltung, und ein Steuermittel zum Abkoppeln der ersten Batterie von der Schaltung sowie zum Ankoppeln der zweiten Batterie an die Schaltung vorzusehen. Ein Kondensator, der parallel zu der ersten Batterie geschaltet ist, wird von dieser während eines ersten und eines zweiten Schaltungszustandes weiter aufgeladen. Dabei ist nur die erste Batterie während des ersten Schaltzustandes an die Schaltung angekoppelt, und nur die zweite Batterie während des zweiten Schaltzustandes an die Schaltung angekoppelt. Am Ende des zweiten Schaltungszustandes wird die zweite Batterie von dem Steuermittel wieder von der Schaltung abgekoppelt und die Parallelschaltung aus der ersten Batterie und dem Kondensator wird zur weiteren Energieversorgung der Schaltung wieder angekoppelt.

Die mit der Erfindung einhergehenden Vorteile liegen insbesondere darin, dass Strom aus der Batterie mit der niedrigen Rate über den Punkt, an dem die Ausgangsspannung der Batterie bei einer normalen Lastbedingung unter den vorgegeben Spannungsschwellwert fällt, hinaus extrahiert und dem Implantat zugeführt werden kann.

Bei einer Ausgestaltung der Erfindung erfolgt die Energieversorgung der Schaltung während des ersten Schaltzustandes durch die Parallelschaltung aus der ersten Batterie und dem Kondensator und während des zweiten Schaltzustandes durch die zweite Batterie. Der erste Schaltzustand endet und der zweite Schaltzustand beginnt dabei, wenn die Spannung an der Parallelschaltung den vorgegebenen Spannungsschwellwert unterschreitet. Damit wird sichergestellt, dass die Spannung an der Parallelschaltung mindestens einen Pegel aufweist, welcher zur Versorgung der Schaltung benötigt wird.

Bei einer weiteren Ausgestaltung der Erfindung endet der zweite Schaltungszustand und der erste Schaltzustand setzt wieder ein, wenn die Spannung an der Parallelschaltung wieder regeneriert ist und den Schwellenwert um ein vorgebbares Maß überschreitet. Mit anderen Worten wird die zweite Batterie nur solange an die Schaltung gekoppelt, bis sich die Spannung an der Parallelschaltung aus erster Batterie und Kondensator erholt bzw. regeneriert hat und wieder als Spannungsquelle für die Schaltung dienen kann.

Bei einer besonders bevorzugten Ausgestaltung der Erfindung dauert der zweite Schaltungszustand solange an, bis die Spannung an der Parallelschaltung näherungsweise die Batteriespannung oder die Leerlaufspannung der ersten Batterie erreicht. Somit wird sichergestellt, dass die erste Batterie genügend Zeit hat, den Kondensator wieder aufzuladen.

Bei einer weiter bevorzugten Ausgestaltung der Erfindung weist das Steuermittel einen Sensor zum Erfassen der Spannung an der Parallelschaltung in vorgegebenen Abständen auf. Damit wird erreicht, dass der Pegel der Spannung an der Parallelschaltung in vorgegebenen Abständen überwacht wird, so dass das Steuermittel gegebenenfalls wieder die erste Batterie an die Schaltung ankoppeln kann.

Weitere Ausgestaltungen der Erfindung sind Gegenstand der Unteransprüche.

Im Folgenden wird die Erfindung unter Bezugnahme auf die Zeichnung näher erläutert.

Es zeigen:
- Fig. 1: ein prinzipielles Schaltbild zur Steuerung der Energieversorgung eines medizinischen Implantates, und
- Fig. 2: eine graphische Darstellung des Batterie- und Kondensatorstromes.

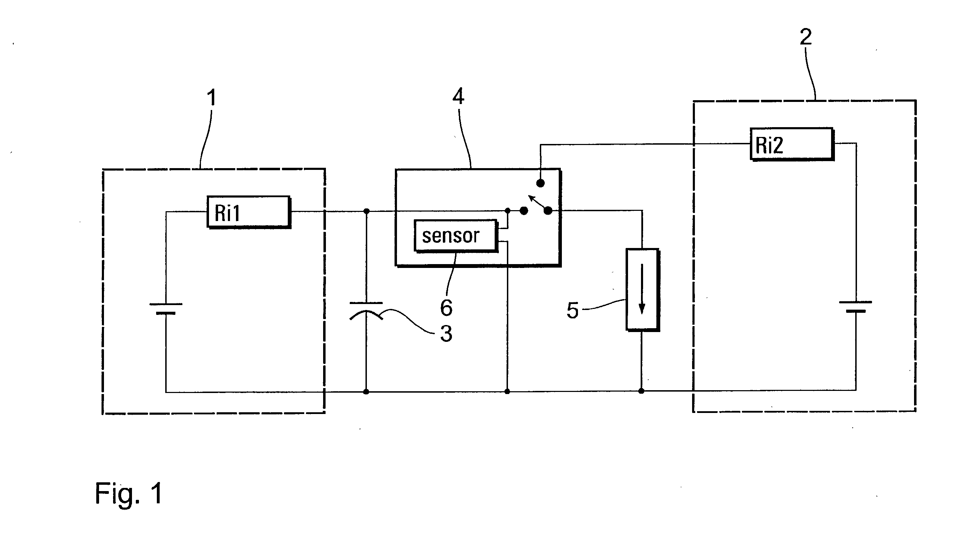

Fig. 1 zeigt ein prinzipielles Schaltbild zur Steuerung der Energieversorgung eines medizinischen Implantates. Das medizinische Implantat weist eine erste Batterie 1, eine zweite Batterie 2, einen Kondensator 3, einen Schalter 4, einen Spannungssensor 6 sowie die herkömmlichen Schaltungskomponenten 5 des medizinischen Implantates auf.

Wenn der Sensor 6 erfasst, dass die Ausgangsspannung der ersten Batterie unter einen ersten vorgegebenen Spannungspegel fällt, wird der Schalter 4 veranlasst, die erste Batterie von der Schaltung 5 abzukoppeln und die zweite Batterie 2 an die Schaltung 5 anzukoppeln, so dass die zweite Batterie die Schaltung 5 mit dem nötigen Strom versorgt. Während dieses zweiten Schaltungszustandes, bei dem die erste Batterie von der Schaltung 5 abgekoppelt ist und nur die zweite Batterie 2 an der Schaltung liegt, erhält der Kondensator 3 den gesamten verfügbaren Strom aus der ersten Batterie 1.

Bei der Stromentnahme aus der ersten Batterie 1 tritt ein anfänglich langsamer, gegen Ende der Entladung schnellerer Spannungsrückgang ein. Wird die Entladung unterbrochen, erhöht sich die Spannung sofort wieder und nimmt bei anschließdender Ruhe wieder langsam zu. Die Batterie "erholt" sich.

Die Höhe der Spannung an der Parallelschaltung aus der ersten Batterie 1 und dem Kondensator 3 wird in vorgegebenen Abständen von dem Sensor 6 überprüft. Sobald diese Spannung wieder dem vorgegebene Spannungspegel entspricht oder ein vorgegebenes Maß über diesem Pegel liegt der zum regulären Betrieb der Schaltung 5 benötigt wird, wird der Schalter 4 veranlasst, die zweite Batterie 2 von der Schaltung 5 abzukoppeln und die Parallelschaltung aus der ersten Batterie 1 und dem Kondensator 3 wieder an die Schaltung 5 zu koppeln, was wiederum den ersten Schaltzustand darstellt. Dabei versorgt der Kondensator zusammen mit der ersten Batterie 1 die Schaltung 5 mit dem benötigten Strom, bis die Spannung an der Parallelschaltung unter den für die Schaltung 5 benötigte Spannungspegel fällt.

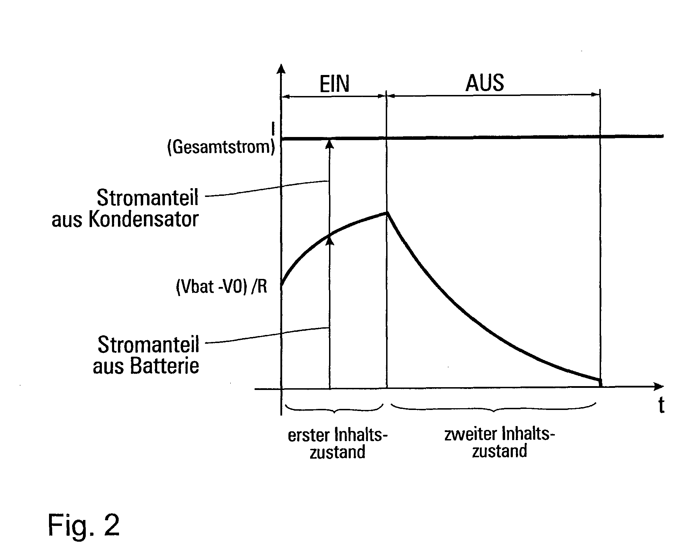

In Fig. 2 sind die von der ersten Batterie 1 und dem Kondensator 3 gelieferten Ströme angezeigt. Vbat entspricht dabei der Leerlaufspannung der ersten Batterie und VO entspricht der Spannung über dem Kondensator am Ende des zweiten Schaltungszustandes. Die Widerstand der Batterie ist mit R bezeichnet. EIN entspricht dem ersten Schaltzustand und AUS dem zweiten Schaltzustand.

Wenn die Batterie sich im Lauf der Zeit erschöpft, wird der erste Schaltungszustand kürzer und der Kondensator 3 ist nicht mehr in der Lage, sich auf die volle Vbat Spannung aufzuladen. Dies hat ebenfalls zur Folge, dass der in Fig. 2 gezeigte anfängliche Strom [(Vbat - VO)/R] kleiner wird, da der Wert des Innenwiderstandes der Batterie R größer wird.

Wenn die Spannung an der Parallelschaltung wieder unter den Spannungsschwellwert sinkt, koppelt der Schalter 4 die Parallelschaltung aus der ersten Batterie 1 und den Kondensator 3 wieder von der Schaltung 5 ab und koppelt die zweite Batterie 2 wieder an die Schaltung 5 an (zweiter Schaltzustand). Während dieser Phase wird der Kondensator wieder auf einen höheren Spannungswert aufgeladen, auf den die erste Batterie 1 sich regeneriert, da während dieses Schaltzustandes die Schaltung, d. h. eine entsprechende Nutzlast nicht angekoppelt ist. Steigt die Spannung an der Parallelschaltung wieder über den vorgegebenen Spannungsschwellwert, d. h. über die zum Versorgen der Schaltung 5 benötigten Spannung, wird die zweite Batterie 2 wieder von der Schaltung 5 abgekoppelt und die Parallelschaltung aus der ersten Batterie 1 und dem Kondensator wird wieder an die Schaltung angekoppelt (erster Schaltzustand).

Während des zweiten Schaltungszustandes, d. h. wenn die Parallelschaltung aus der ersten Batterie 1 und dem Kondensator 3 von der Schaltung 5 abgekoppelt ist, bildet der Innenwiderstand der Batterie 1 und der Kondensator 3 eine RC-Schaltung. Dies bedeutet, dass der Spannungsabfall über dem Innenwiderstand der ersten Batterie abfällt, während der Kondensator 3 durch die Batterie aufgeladen wird, bis die Spannung an dem Kondensator der Leerlaufspannung der ersten Batterie entspricht. Bei voranschreitender Erschöpfung der Batterie wird die Leerlaufspannung der ersten Batterie geringer sein als ihre ursprüngliche Leerlaufspannung. Dies hat zur Folge, dass der Kondensator somit auch nicht mehr auf die anfängliche Leerlaufspannung der ersten Batterie 1 aufgeladen werden kann. Irgendwann ist die Erschöpfung der Batterie so groß sein, dass die durch Aufladung des Kondensators 3 erhaltene Spannung geringer ist, als die für die Schaltung 5 benötigte Spannung.

Im Prinzip wird durch die in Fig. 1 gezeigte Schaltung eine Ladungspumpe realisiert, die versucht die in der ersten Batterie verbleibende Ladungskapazität über den Punkt hinaus zu extrahieren, an dem die erste Batterie 1 den für die Versorgung der Schaltung 5 benötigten Strom kontinuierlich zuführt.

Der Kondensator 3 wird dabei relativ groß gewählt und besitzt bevorzugt eine Kapazität von 68 Mikrofarad.

## Patentansprüche

1. Elektrisch aktives medizinisches Implantat, mit einer Schaltung (5), mindestens einer ersten Batterie (1) zum Abgeben eines niedrigen Stromes und einer zweiten Batterie (2) zum Abgeben eines hohen Stromes an die Schaltung (5) und einem Steuermittel (4) zum Abkoppeln der ersten Batterie (1) von der Schaltung (5) und zum Ankoppeln der zweiten Batterie (2) an die Schaltung (5), **gekennzeichnet durch** einen parallel zu der ersten Batterie (1) geschalteten Kondensator (3), der von der ersten Batterie sowohl während eines ersten Schaltzustandes, während dem die erste Batterie an die Schaltung (5) angekoppelt ist als auch während eines zweiten Schaltzustandes, während dem die Schaltung (5) nur an der zweiten Batterie (1) angekoppelt ist, aufladbar ist, wobei das Steuermittel (4) die zweite Batterie (2) am Ende des zweiten Schaltungszustandes wieder von der Schaltung (5) abkoppelt und die Parallelschaltung aus der ersten Batterie (1) und dem Kondensator (3) zur weiteren Energieversorgung wieder an die Schaltung (5) ankoppelt.

2. Medizinisches Implantat nach Anspruch 1, **dadurch gekennzeichnet, dass** der erste Schaltungszustand, während dem die Energieversorgung der Schaltung (5) durch die Parallelschaltung aus der ersten Batterie (1) und dem Kondensator (3) erfolgt, endet und der zweite Schaltungszustand, während dem die Energieversorgung der Schaltung (5) durch die zweite Batterie erfolgt, beginnt, wenn die Spannung der Parallelschaltung aus der ersten Batterie (1) und dem Kondensator (3) den vorgegebenen Spannungsschwellwert unterschreitet.

3. Medizinisches Implantat nach einem der vorherigen Ansprüche, **dadurch gekennzeichnet, dass** der zweite Schaltungszustand endet und der erste Schaltungszustand wieder einsetzt, wenn die Spannung an der Parallelschaltung den Schwellenwert um ein vorgebbares Maß überschreitet.

4. Medizinisches Implantat nach Anspruch 3, **dadurch gekennzeichnet, dass** der zweite Schaltungszustand solange andauert, bis die Spannung an der Parallelschaltung näherungsweise die Batteriespannung oder die Leerlaufspannung der ersten Batterie (1) erreicht.

5. Medizinisches Implantat nach einem der vorstehenden Ansprüche, **dadurch gekennzeichnet, dass** das Steuermittel (4) die Schaltung (5) wiederholt von dem ersten Schaltzustand in den zweiten Schaltzustand, anschließend wieder in den ersten Schaltzustand und dann wieder in den zweiten Schaltzustand etc. schaltet.

6. Medizinisches Implantat nach einem der vorherigen Ansprüche, **dadurch gekennzeichnet, dass** das Steuermittel (4) einen Sensor (6) zum Erfassen der Spannung an der Parallelschaltung aus der ersten Batterie (1) und dem Kondensator in vorgegebenen Abständen aufweist.

7. Medizinisches Implantat nach einem der vorherigen Ansprüche, **dadurch gekennzeichnet, dass** das Steuermittel (4) einen Schalter zum An- und Abkoppeln der ersten und zweiten Batterie (1, 2) von der Schaltung (5) aufweist.

8. Medizinisches Implantat nach einem der vorherigen Ansprüche, **dadurch gekennzeichnet, dass** der Kondensator (3) mehrere µF, insbesondere mehrere 68 µF, groß ist.

9. Medizinisches Implantat nach einem der vorherigen Ansprüche, **dadurch gekennzeichnet, dass** die erste Batterie (1) als Batterie mit geringer Leistung aber mit einer hohen Speicherkapazität ausgestaltet ist.

10. Medizinisches Implantat nach einem der vorherigen Ansprüche, **dadurch gekennzeichnet, dass** die zweite Batterie (2) als Batterie mit hoher Leistung aber mit einer geringen Speicherkapazität ausgestaltet ist.
